# EUROPÄISCHE PATENTSCHRIFT

(11) **EP 1 870 055 B1**
(45) Veröffentlichungstag und Bekanntmachung des Hinweises auf die Patenterteilung: **06.11.2019**
(21) Anmeldenummer: 07012274.2
(22) Anmeldetag: 22.06.2007
(51) Int. Cl.: A61D 1/02, A61M 5/50

(54) **Originalitätsssicherer Injektor oder Applikator zur Absage eines flüssigen oder pastösen Arzneimittels**
Tamper-proof injector or applicator for dispensing a liquid or paste-like drug
Injecteur ou applicateur inviolable destiné à distribuer un médicament liquide ou pâteux

(30) Priorität: 22.06.2006 DE 102006028678; 13.09.2006 DE 102006043033
(43) Veröffentlichungstag der Anmeldung: 26.12.2007
(73) Patentinhaber: elm-plastic GmbH, 54647 Dudeldorf (DE)
(72) Erfinder: Lonien, Birgit, 54647 Dudeldorf (DE); Möhs, Sascha, 54647 Dudeldorf (DE)
(74) Vertreter: Zinnecker, Armin

(56) Entgegenhaltungen:
- EP-A- 0 830 868
- EP-A- 1 477 128
- WO-A-93/02728
- DE-A1-102004 055 453
- DE-U1-202005 011 806
- US-B1- 6 402 721

## Beschreibung

Die Erfindung betrifft einen originalitätssicheren (tamper-proof) Injektor oder Applikator zur Abgabe eines flüssigen oder pastösen Arzneimittels nach dem Oberbegriff des Anspruchs 1. Der Injektor oder Applikator dient insbesondere zur Abgabe eines Arzneimittels für die ärztliche Behandlung eines Tieres, insbesondere für die ärztliche Behandlung des Euters eines Tieres. Darüber hinaus kann die Erfindung für jede bekannte Darreichungsform (oral, nasal etc.) Anwendung finden.

EP 1 477 128 A1 zeigt einen Injektor mit einem Gehäuseteil, einem Kolbenkörper und einer Verschlußkappe. Die Verschlußkappe umfaßt ein Kappenteil zum Verschließen der Öffnung des Injektors und ein Verbindungsteil zum Verbinden der Verschlußkappe mit dem Injektor. Das Kappenteil und das Verbindungsteil sind durch eine Abreißkante lösbar verbunden. Hierdurch soll eine Originalitätssicherung geschaffen werden. Durch den Injektor nach der EP 1 477 128 A1 wird allerdings lediglich eine Manipulation im Bereich der Kappe verhindert. Manipulationen im Bereich des Kolbens werden nicht ausgeschlossen.

Druckschriften EP 830 868 A1 und DE 10 2004 055453 A1 zeigen jeweils Injektoren gemäß dem Oberbegriff von Anspruch 1.

Weitere Injektoren sind aus den Druckschriften WO 93/02728 A1, DE 20 2005 011806 U1 und US 6 402 721 B1 bekannt.

Hiervon ausgehend liegt der Erfindung die Aufgabe zugrunde, einen Injektor oder Applikator der eingangs angegebenen Art mit einer verbesserten Originalitätssicherung vorzuschlagen.

Erfindungsgemäß wird diese Aufgabe durch die kennzeichnenden Merkmale des Anspruchs 1 gelöst.

Das Gehäuseteil weist eine Hinterschneidung auf. Der Kolbenkörper weist einen Vorsprung auf. Die Hinterschneidung und der Vorsprung sind derart aufeinander abgestimmt, daß der Kolbenkörper oder ein Teil davon nicht aus dem Gehäuseteil herausgezogen werden kann. Der Kolbenkörper oder ein Teil davon wird beim Versuch des Herausziehens aus dem Gehäuseteil an der Hinterschneidung festgehalten. Damit weist der Kolbenkörper erfindungsgemäß eine Sicherung gegen ein Herausziehen aus dem Gehäuseteil auf. Ein Austausch oder eine Manipulation der in dem Gehäuseteil vorhandenen Flüssigkeit oder Paste, der ein vollständiges Herausziehen des Kolbenkörpers aus dem Gehäuseteil voraussetzt, ist nicht möglich. Hierdurch werden auch Manipulationen im Bereich des Kolbens verhindert. Durch die Erfindung wird ein verbesserter Originalitätsverschluß für einen Injektor oder Applikator zur Abgabe eines flüssigen oder pastösen Arzneimittels geschaffen. Der erfindungsgemäße Injektor oder Applikator ist "tamper proof" bzw. "tamper evident" ausgestaltet.

Erfindungsgemäß umfaßt der Kolbenkörper einen Stößel und einen Kolben, der mit dem Stößel lösbar verbunden ist. In diesem Fall wird der Vorsprung des Kolbenkörpers von dem Kolben gebildet. Wenn der Kolbenkörper aus dem Gehäuseteil herausgezogen wird, stößt der Kolben an der Hinterschneidung des Gehäuseteils an. Wenn dann versucht wird, den Kolbenkörper gewaltsam aus dem Gehäuseteil herauszuziehen, bleibt der Kolben an der Hinterschneidung hängen. Der Stößel und der Kolben sind derart miteinander verbunden, daß in diesem Fall der Kolben von dem Stößel getrennt wird, so daß der Stößel vollständig aus dem Gehäuseteil herausgezogen werden kann, der Kolben jedoch in dem Gehäuseteil verbleibt, wo er eine Originalitätssicherung bildet und eine Manipulation des in dem Kolbenkörper befindlichen flüssigen oder pastösen Arzneimittels verhindert.

Vorteilhafte Weiterbildungen sind in den Unteransprüchen beschrieben.

Das Gehäuseteil kann eine oder mehrere Lamellen und/oder einen oder mehrere Haken aufweisen. Die Lamellen und/oder Haken sind derart ausgestaltet, daß sie eine Hinterschneidung bilden und ein Herausziehen des Kolbenkörpers aus dem Gehäuseteil verhindern.

Wenn der Kolbenkörper einen Stößel und einen Kolben umfaßt, die lösbar miteinander verbunden sind, können der Stößel und der Kolben aus einem Stück oder aus zwei Stücken hergestellt sein. Insbesondere können der Stößel und der Kolben durch eine Sollbruchstelle miteinander verbunden sein. Anstelle einer Sollbruchstelle oder zusätzlich zu dieser können der Stößel und der Kolben durch ein Siegelband miteinander verbunden sein.

Vorteilhaft ist es, wenn das Verbindungsteil ein Klemmteil aufweist. Das Verbindungsteil kann dadurch mit dem Injektor oder Applikator verklemmt werden.

Vorzugsweise weist das Verbindungsteil oder Klemmteil eine Wulst auf. Die Wulst ist vorzugsweise derart ausgestaltet, daß sie einen entsprechenden Hohlraum, der im Injektor oder Applikator vorgesehen ist, insbesondere eine Nut, hintergreift.

Nach einer weiteren vorteilhaften Weiterbildung ist das Verbindungsteil mit dem Injektor oder Applikator verpresst. Insbesondere kann das Verbindungsteil mit dem Injektor oder Applikator kraftschlüssig verbunden sein. Hierdurch kann auf einfache Weise gewährleistet werden, daß das Verbindungsteil mit dem Injektor oder Applikator verbunden bleibt, wenn das Kappenteil gelöst bzw. abgerissen wird.

Nach einer weiteren vorteilhaften Weiterbildung ist das Verbindungsteil mit dem Injektor oder Applikator verschweißt.

Eine weitere vorteilhafte Weiterbildung ist dadurch gekennzeichnet, daß das Verbindungsteil mit dem Injektor oder Applikator formschlüssig verbunden ist.

Die Abreißkante, die das Kappenteil und das Verbindungsteil verbindet, verläuft vorzugsweise in radialer Richtung.

Vorteilhaft ist es, wenn das Kappenteil einen Dichtzapfen aufweist. Der Dichtzapfen ist vorzugsweise derart ausgestaltet, daß er in die Öffnung der Spitze des Gehäuseteils eingreift und diese dichtend verschließt, wenn die Verschlußkappe mit dem Injektor oder Applikator verbunden und das Kappenteil noch nicht von dem Verbindungsteil gelöst ist.

Ein Ausführungsbeispiel der Erfindung wird nachstehend anhand der beigefügten Zeichnung im einzelnen erläutert. In der Zeichnung zeigt
- Fig. 1: einen Injektor mit aufgesetzter Verschlußkappe in einer seitlichen Schnittansicht,
- Fig. 1a,b,c: vergrößerte Teilansichten der Fig. 1,
- Fig. 2: das obere Ende des Injektors und die Verschlußkappe in einer vergrößerten Darstellung,
- Fig. 2a: eine vergrößerte Teilansicht der Fig. 2,
- Fig. 3: die Verschlußkappe nach der Ablösung des Kappenteils,
- Fig. 4: einen nicht beanspruchten Injektor mit einem einteiligen Kolbenkörper, der teilweise aus dem Gehäuseteil herausgezogen ist,
- Fig. 5: den Injektor mit einem Kolbenkörper, der einen Stößel und einen Kolben umfaßt und der teilweise aus dem Gehäuseteil herausgezogen ist,
- Fig. 6: den Injektor gemäß Fig. 5 mit ganz herausgezogenem Stößel und
- Fig. 7: den hinteren Bereich des Gehäuseteils in einer vergrö-ßerten Darstellung.

Der in der Zeichnung gezeigte Injektor 4, der auch als Injektionsspritze bezeichnet werden kann, umfaßt ein Gehäuseteil 1 und einen darin längsverschieblich gelagerten Kolbenkörper 2. Am vorderen Ende des Gehäuseteils 1 ist eine Spitze 5 vorgesehen, die mit dem Gehäuseteil 1 einstückig ist. Innerhalb der Spitze 5 befindet sich ein Kanal 6, der mit dem Hohlraum 7 zwischen den Kolbenkörper 2 und dem Gehäuseteil 1 verbunden ist und der am äußeren Ende der Spitze 5 in eine Öffnung 8 mündet.

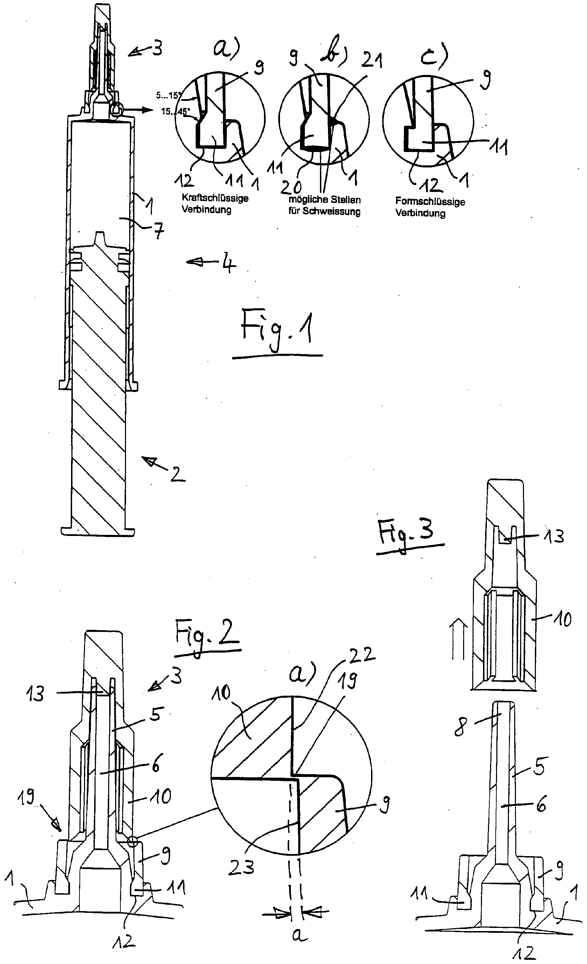

Die Verschlußkappe 3 umfaßt, wie insbesondere in Fig. 1, 2 und 3 dargestellt, ein Verbindungsteil 9 und ein Kappenteil 10. Das Verbindungsteil 9 weist an seinem unteren Ende eine umlaufende Wulst 11 auf, die eine entsprechende Nut 12 in dem Gehäuseteil 1 hintergreift. Die Nut 12 ist im Bereich des vorderen Endes des Gehäuseteils 1 an dessen Außenseite vorgesehen. Sie umläuft die Spitze 5 in ihrem Fußbereich.

Bei der in Fig. 1a gezeigten Ausführungsform ist die Wulst 11 mit der Nut 12 kraftschlüssig verbunden. Die Nut 12 ist in ihrem Querschnitt über einen Bereich von mehr als 180° geschlossen, so daß die Wulst 11 in ihr festgehalten wird. Der Querschnitt der Nut 12 verläuft im wesentlichen U-förmig. Der radial äußere Schenkel des U-förmigen Profils der Nut 12 verläuft geradlinig nach oben. Der radial innere Schenkel weist in seinem oberen Endbereich eine Schräge auf, die zur Mitte der Nut 12 hin verläuft. Der Winkel dieser Schräge beträgt 15 bis 45°. Der daran anschließende Bereich des Verbindungsteils 9 verläuft in einem Winkel von 5 bis 15° von der Nut 12 weg. Die Wulst 11 verläuft zur Nut 12 komplementär. Sie wird in die Nut 12 eingebracht und mit der Nut verklemmt oder verpresst, wodurch das Verbindungsteil 9 fest auf dem Gehäuseteil 1 fixiert wird.

Bei der in Fig. 1b gezeigten Variante ist die Wulst 11 mit dem Verbindungsteil 9 verschweißt. Die erste Verschweißung 20 befindet sich am Grund der Nut 12. Die zweite Verschweißung 21 befindet sich am oberen Ende des äußeren Schenkels des U-förmigen Profils der Nut 12. Es ist möglich, beide Verschweißungen 20, 21 vorzusehen oder nur eine davon. Im übrigen entsprechen die Formgestaltungen der Wulst 11 und der Nut 12 bei der Ausführungsform nach Fig. 1b derjenigen nach Fig. 1a. Die Verschweißungen 20 und/oder 21 können zu der kraftschlüssigen Verbindung gemäß Fig. 1a hinzutreten. Es ist allerdings auch möglich, daß die Verbindungskräfte überwiegend oder ausschließlich durch eine oder beide Verschweißungen 20, 21 bewirkt werden. Ferner kann im Falle einer oder mehrerer Verschweißungen 20, 21 auf eine Hinterschneidung der Wulst 11 in der Nut 12 verzichtet werden.

Bei der Ausführungsform nach Fig. 1c ist die Wulst 11 mit der Nut 12 formschlüssig verbunden. Hier beträgt der Winkel der Schräge nicht, wie bei Fig. 1a, 15 bis 45°, sondern 90°.

Das Kappenteil 10 weist einen Dichtzapfen 13 auf, der sich im Inneren des oberen Endbereichs des Kappenteils 10 befindet und der nach unten gerichtet ist. Wenn die Verschlußkappe 3 mit dem Gehäuseteil 1 verbunden ist greift der Dichtzapfen 13 in die Öffnung 8 der Spitze 5 des Gehäuseteils 1 ein, wie insbesondere aus Fig. 2 ersichtlich. Die Öffnung 8 wird durch den Dichtzapfen 13 dichtend verschlossen.

Das Verbindungsteil 9 und das Kappenteil 10 sind durch eine Abreißkante 19 miteinander verbunden. Die Abreißkante wird durch einen umlaufenden Ringbereich geringen Querschnitts gebildet. Sie verbindet den oberen Endbereich des Verbindungsteils 9 mit dem unteren Endbereich des Kappenteils 10. Verbindungsteil 9, Kappenteil 10 und Abreißkante 19 sind einstückig. Wie insbesondere aus Fig. 2a ersichtlich verläuft die Abreißkante 19 in radialer Richtung. In der in Fig. 3 gezeigten Profildarstellung verläuft die äußere Profilkante 22 des Kappenteils 10 in radialer Richtung weiter innen als die innere Kante 23 des Verbindungsteils 9. Der hierdurch entstehende Versatz a wird durch die horizontal verlaufende Abreißkante 19 überbrückt. Die im Profil horizontalen Begrenzungen der Abreißkante 19 verlaufen parallel. Die Abreißkante 19 ist als schmaler Steg ausgebildet. Sie ist gegenüber der Abreißkante nach der EP 1 477 128 A1 einfacher und materialsparender ausgestaltet.

Wenn der Injektor zur Abgabe der in dem Hohlraum 7 befindlichen Flüssigkeit oder Paste benutzt werden soll, muß das Kappenteil 10 entfernt werden. Dies erfolgt dadurch, daß das Kappenteil 10 abgerissen wird. Dabei löst sich das Kappenteil 10 im Bereich der Abreißkante 19 von dem Verbindungsteil 9. Das Kappenteil 10 kann nach oben entfernt werden, wie in Fig. 3 gezeigt, so daß die Spitze 5 freigelegt wird. Das Verbindungsteil 9 bleibt aufgrund der Verklemmung oder Verpressung der Wulst 11 in der Nut 12 mit dem Gehäuseteil 1 verbunden. Hierdurch ist es unmöglich, in die Nut 12 des Gehäuseteils 1 eine weitere, unversehrte Verschlußkappe 3 einzusetzen. Das fest mit dem Gehäuseteil 1 verbunden bleibende Verbindungsteil 9 bildet auf diese Weise eine Originalitätssicherung.

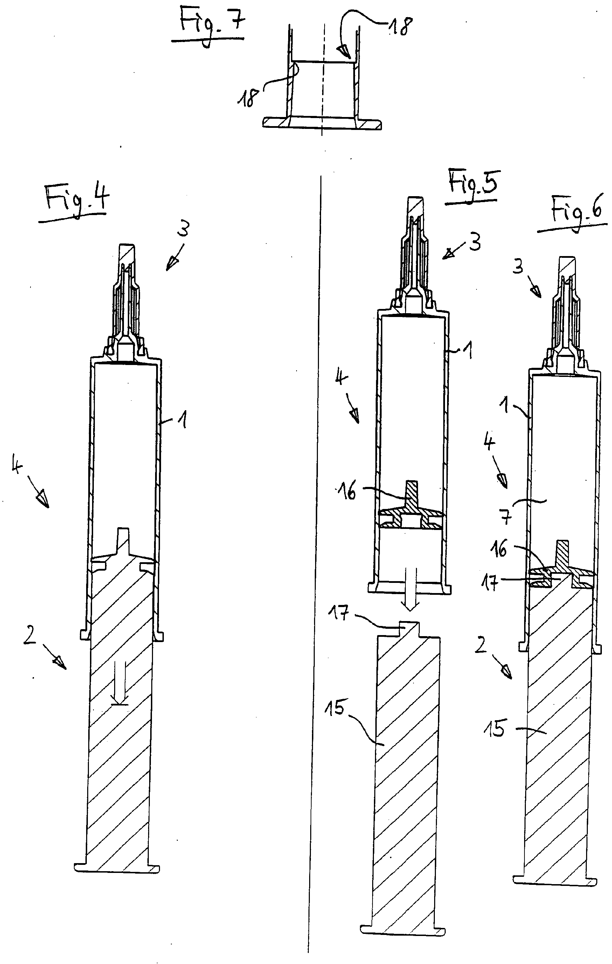

Bei der in Fig. 5 und 6 gezeigten Ausführungsform besteht der Kolbenkörper 2 aus einem Stößel 15, der hinten aus dem Gehäuseteil 1 herausragt, und einem Kolben 16, der dem vorderen Teil des Gehäuseteils 1 zugewandt ist. Der Hohlraum 7 wird von dem Gehäuseteil 1 und der vorderen Endfläche des Kolbens 16 umschlossen. Stößel 15 und Kolben 16 sind durch einen leichten Kraftschluß miteinander verbunden. Zu diesem Zweck weist der Stößel 15 an seinem vorderen Ende eine Erhebung 17 auf, die in eine entsprechende Vertiefung an der Rückseite des Kolbens 16 eingreift, Dort sind der Stößel 15 und der Kolben 16 miteinander verklemmt.

Das Gehäuseteil 1 weist im Bereich seines hinteren Endes eine Hinterschneidung 18 auf. Die Hinterschneidung 18 wird von einer nach innen weisenden Stufe gebildet. Diese Stufe kann umlaufend ausgebildet sein. Es können allerdings auch mehrere Stufen über den Umfang verteilt sein. Der Innendurchmesser des Gehäuseteils 1 bzw. der Abstand gegenüberliegender Stufen ist im hinteren Bereich des Gehäuseteils 1 geringer als im weiter vorne liegenden Bereich. Ferner ist dieser Durchmesser bzw. sind diese Abstände auf den Kolben 16 derart abgestimmt, daß dieser Kolben 16 in das Gehäuseteil 1 hineinbewegt werden kann, jedoch nicht mehr aus dem Gehäuseteil 1 herausbewegt werden kann. Wenn der Kolbenkörper 2 aus dem Gehäuseteil 1 herausgezogen wird, wird der Kolben 16 von der Hinterschneidung 18 zurückgehalten. Beim weiteren Zug nach hinten löst sich der Stößel 15 vom Kolben 16, wie in Fig. 5 gezeigt. Der Kolben 16 bildet in dieser Stellung eine Originalitätssicherung, die es verhindert, daß die in dem Hohlraum 7 befindliche Flüssigkeit oder Paste manipuliert oder durch eine andere Flüssigkeit oder Paste ersetzt werden kann.

Anstelle der Hinterschneidung 18 können eine oder mehrere Lamellen und/oder Haken vorgesehen sein. Auch diese Teile müssen auf den Kolben 16 in der Weise abgestimmt sein, daß er durch diese Teile an einer Herausbewegung aus dem Gehäuseteil 1 gehindert wird.

Es wäre auch möglich, den Stößel 15 und den Kolben 16 durch ein Siegelband oder ein ähnliches Bauteil miteinander zu verbinden, welches beim Versuch des Herausziehens des Kolbens 16 aus dem Gehäuseteil 1 abreißt. Das Siegelband kann beim Spritzgießen des Kolbenkörpers 2 hergestellt werden.

Eine weitere Möglichkeit besteht darin, den Stößel 15 und den Kolben 16 einstückig herzustellen und durch eine Sollbruchstelle miteinander zu verbinden.

Bei der in Fig. 4 gezeigten, nicht beanspruchten Ausführungsform ist der Kolbenkörper 2 einteilig. Hierzu können der Stößel 15 und der Kolben 16 einstückig hergestellt sein. Der Stößel 15 und der Kolben 16 können allerdings auch zweistückig hergestellt und unlösbar miteinander verbunden sein. In beiden Fällen würde der gesamte, aus Stößel 15 und Kolben 16 bestehende Kolbenkörper 2 durch die Hinterschneidung 18 oder ein ähnliches Bauteil am Herausziehen aus dem Gehäuseteil 1 gehindert werden.

In den Zeichnungsfiguren sind ein Gehäuseteil 1 und ein Kolbenkörper 2 mit kreisrundem Querschnitt gezeigt. Es sind jedoch auch andere Querschnittsformen möglich, beispielsweise eine Kreuzform oder eine andere Form.

Eine weitere Sicherungsmöglichkeit besteht darin, in dem Stößel 15 eine Einbuchtung oder eine Ausbuchtung vorzusehen, die mit der Hinterschneidung oder einem ähnlichen Teil des Gehäuseteils 1 derart zusammenwirkt, daß ein Herausziehen des Stößels 15 aus dem Gehäuseteil 1 nicht mehr möglich ist.

Durch die Erfindung wird eine Originalitätssicherung für einen vorgefüllten Injektor oder Applikator geschaffen, die verhindert, daß die eingefüllte Flüssigkeit oder Paste manipuliert oder eine andere als die ursprünglich eingefüllte Flüssigkeit oder Paste angewendet werden kann.

In den Zeichnungen ist ein Injektor zur Abgabe eines flüssigen oder pastösen Arzneimittels gezeigt. Die Erfindung ist allerdings auch bei einem Applikator zur Abgabe eines flüssigen oder pastösen Arzneimittels anwendbar.

## Patentansprüche

1. Injektor oder Applikator zur Abgabe eines flüssigen oder pastösen Arzneimittels mit einem Gehäuseteil (1), einem darin längsverschieblich gelagerten Kolbenkörper (2) und einer Verschlußkappe (3) mit einem Kappenteil (10) zum Verschließen der Öffnung (8) des Injektors oder Applikators und mit einem Verbindungsteil (9) zum Verbinden der Verschlußkappe (3) mit dem Gehäuseteil (1), wobei das Kappenteil (10) und das Verbindungsteil (9) durch eine Abreißkante (19) lösbar verbunden sind, wobei das Gehäuseteil (1) eine Hinterschneidung (18) aufweist und daß der Kolbenkörper (2) einen Vorsprung aufweist, die derart aufeinander abgestimmt sind, dass der Kolbenkörper (2) oder ein Teil davon beim Versuch des Herausziehens aus dem Gehäuseteil (1) an der Hinterschneidung (18) festgehalten wird,
**dadurch gekennzeichnet,**
**daß** der Kolbenkörper (2) einen Stößel (15) und einen Kolben (16) umfaßt, der mit dem Stößel (15) lösbar verbunden ist.

2. Injektor oder Applikator nach Anspruch 1, **dadurch gekennzeichnet, daß** das Gehäuseteil (1) eine oder mehrere Lamellen und/oder Haken aufweist.

3. Injektor oder Applikator nach Anspruch 1 oder 2, **dadurch gekennzeichnet, daß** der Kolbenkörper (2) einteilig ist.

4. Injektor oder Applikator nach einem der vorhergehenden Ansprüche, **dadurch gekennzeichnet, daß** der Stößel (15) und der Kolben (16) durch ein Siegelband und/oder durch eine Sollbruchstelle miteinander verbunden sind.

5. Injektor oder Applikator nach einem der vorhergehenden Ansprüche, **dadurch gekennzeichnet, daß** das Verbindungsteil (9) ein Klemmteil aufweist.

6. Injektor oder Applikator nach einem der vorhergehenden Ansprüche, **dadurch gekennzeichnet, daß** das Verbindungsteil (9) eine Wulst (11) aufweist.

7. Injektor oder Applikator nach einem der vorhergehenden Ansprüche, **dadurch gekennzeichnet, daß** das Verbindungsteil (9) mit dem Injektor oder Applikator verpresst ist.

8. Injektor oder Applikator nach einem der vorhergehenden Ansprüche, **dadurch gekennzeichnet, daß** das Verbindungsteil (9) mit dem Injektor oder Applikator verschweißt (20, 21) ist.

9. Injektor oder Applikator nach einem der vorhergehenden Ansprüche, **dadurch gekennzeichnet, daß** das Verbindungsteil (9) mit dem Injektor oder Applikator formschlüssig verbunden ist.

10. Injektor oder Applikator nach einem der vorhergehenden Ansprüche, **dadurch gekennzeichnet, daß** die das Kappenteil (10) und das Verbindungsteil (9) verbindende Abreißkante (19) in radialer Richtung verläuft.

11. Injektor oder Applikator nach einem der vorhergehenden Ansprüche, **dadurch gekennzeichnet, daß** das Kappenteil (10) einen Dichtzapfen (13) aufweist.

## Claims

1. An injector or applicator for dispensing a liquid or pasty drug, comprising a housing portion (1), a piston body (2) supported therein in a longitudinally-displaceable manner, and a closing cap (3) with a cap portion (10) for closing the opening (8) of the injector or applicator and with a connecting portion (9) for connecting the closing cap (3) with the housing portion (1), the cap portion (10) and the connecting portion (9) being releasably connected by a tear edge (19), wherein the housing portion (1) comprises an undercut (18) and in that the piston body (2) comprises a protrusion, which are adjusted to one another in such a way that the piston body (2) or a part thereof are retained at the undercut (18) when trying to withdraw the same from the housing portion (1),
**characterized in that**
the piston body (2) comprises a plunger (15) and a piston (16) which is releasably connected with the plunger (15).

2. The injector or applicator according to claim 1, **characterized in that** the housing portion (1) has one or more lamellae and/or hooks.

3. The injector or applicator according to claim 1 or 2, **characterized in that** the piston body (2) is made of one piece.

4. The injector or applicator according to any of the preceding claims, **characterized in that** the plunger (15) and the piston (16) are connected with each other by a sealing tape and/or by a predetermined breaking point.

5. The injector or applicator according to any of the preceding claims, **characterized in that** the connecting portion (9) has a clamping portion.

6. The injector or applicator according to any of the preceding claims, **characterized in that** the connecting portion (9) has a bead (11).

7. The injector or applicator according to any of the preceding claims, **characterized in that** the connecting portion (9) is pressed into the injector or applicator.

8. The injector or applicator according to any of the preceding claims, **characterized in that** the connecting portion (9) is welded to the injector or applicator (20, 21).

9. The injector or applicator according to any of the preceding claims, **characterized in that** the connecting portion (9) is positively connected with the injector or applicator.

10. The injector or applicator according to any of the preceding claims, **characterized in that** the tear edge (19) connecting the cap portion (10) and the connecting portion (9) extends in radial direction.

11. The injector or applicator according to any of the preceding claims, **characterized in that** the cap portion (10) has a sealing pin (13).

## Revendications

1. Injecteur ou applicateur destiné à distribuer un médicament liquide ou pâteux, comprenant une partie formant boîtier (1), un corps de piston (2) monté déplaçable longitudinalement dans celle-ci et un capuchon de fermeture (3) doté d'une partie capuchon (10) destinée à fermer l'ouverture (8) de l'injecteur ou l'applicateur et d'une partie de liaison (9) destinée à relier le capuchon de fermeture (3) à la partie formant boîtier (1), la partie capuchon (10) et l'élément de liaison (9) étant reliés de manière détachable par un bord de déchirement (19), la partie formant boîtier (1) comportant une contre-dépouille (18) et en ce que le corps de piston (2) comporte une saillie, lesquelles concordent l'une avec l'autre de telle manière que le corps de piston (2) ou une partie de celui-ci est retenu(e) sur la contre-dépouille (18) lors d'une tentative d'extraction hors de la partie formant boîtier (1),
**caractérisé en ce que**
le corps de piston (2) comprend un poussoir (15) et un piston (16), qui est relié de manière détachable au poussoir (15).

2. Injecteur ou applicateur selon la revendication 1, **caractérisé en ce que** la partie formant boîtier (1) comporte un(e) ou plusieurs lamelles et/ou crochets.

3. Injecteur ou applicateur selon la revendication 1 ou 2, **caractérisé en ce que** le corps de piston (2) est en une seule partie.

4. Injecteur ou applicateur selon l'une des revendications précédentes, **caractérisé en ce que** le poussoir (15) et le piston (16) sont reliés l'un à l'autre par une bande de scellé et/ou par un point destiné à la rupture.

5. Injecteur ou applicateur selon l'une des revendications précédentes, **caractérisé en ce que** l'élément de liaison (9) comporte un élément de serrage.

6. Injecteur ou applicateur selon l'une des revendications précédentes, **caractérisé en ce que** l'élément de liaison (9) comporte un renflement (11).

7. Injecteur ou applicateur selon l'une des revendications précédentes, **caractérisé en ce que** l'élément de liaison (9) est comprimé avec l'injecteur ou l'applicateur.

8. Injecteur ou applicateur selon l'une des revendications précédentes, **caractérisé en ce que** l'élément de liaison (9) est soudé (20, 21) avec l'injecteur ou l'applicateur.

9. Injecteur ou applicateur selon l'une des revendications précédentes, **caractérisé en ce que** l'élément de liaison (9) est relié par complémentarité de forme à l'injecteur ou l'applicateur.

10. Injecteur ou applicateur selon l'une des revendications précédentes, **caractérisé en ce que** le rebord de déchirement (19) reliant la partie capuchon (10) et l'élément de liaison (9) s'étend dans la direction radiale.

11. Injecteur ou applicateur selon l'une des revendications précédentes, **caractérisé en ce que** la partie capuchon (10) comporte un doigt d'étanchéité (13).
